(19) Europäisches Patentamt
European Patent Office
Office européen des brevets

(11) Publication number: **0 128 009**
**B1**

(12) **EUROPEAN PATENT SPECIFICATION**

(45) Date of publication of patent specification: **09.08.89**

(51) Int. Cl.⁴: **A 61 K 45/02** // (A61K45/02, 37:02)

(21) Application number: **84303652.6**

(22) Date of filing: **31.05.84**

(54) **Synergistic composition comprising gamma interferon and lymphotoxin, and its preparation.**

(30) Priority: **01.06.83 US 499952**

(43) Date of publication of application:
**12.12.84 Bulletin 84/50**

(45) Publication of the grant of the patent:
**09.08.89 Bulletin 89/32**

(84) Designated Contracting States:
**AT BE CH DE FR GB IT LI LU NL SE**

(56) References cited:
**DE-A-3 227 262**

**JOURNAL OF IMMUNOLOGY, vol. 130, no. 2, February 1983, pages 518-520, American Association of Immunologists, US; T.W. WILLIAMS et al.: "In vitro synergism between interferons and human lymphotoxin: Enhancement of lymphotxin-induced target cell killing"**

(73) Proprietor: **GENENTECH, INC.**
**460 Point San Bruno Boulevard**
**South San Francisco California 94080 (US)**

(72) Inventor: **Aggarwal, Bharat Bhushan**
**324 Del Rosa Way**
**San Mateo California 94403 (US)**
Inventor: **Lee, Sang He**
**1035 San Carlos Avenue**
**El Granada California 94018 (US)**

(74) Representative: **Armitage, Ian Michael et al**
**MEWBURN ELLIS & CO. 2/3 Cursitor Street**
**London EC4A 1BQ (GB)**

**EP 0 128 009 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background

The present invention relates to the field of inhibition of tumor-cell growth, specifically using cytotoxic and cytolytic agents. In particular, the present invention relates to the combined use of lymphotoxin and gamma interferon in such inhibition.

Lymphotoxin has been implicated in the regulation of the immune system and has been reported to inhibit tumor cell growth both *in vivo* (see, e.g. Kahn, A. *et al. Hematology and Oncology*, 11: 128a (1981)) and *in vitro* (see e.g. Gately, M.K. *et al. Immunol.*, 27: B2 (1976)). Under *in vitro* conditions, it is a more potent inhibitor of tumor cells than of normal ones from the same species (see e.g., Evans, C.H. *et al. Cancer Res.* 35: 1035 (1975)). Lymphotoxin may be assayed by its cytotoxic and cytolytic effects, as well as its capacity to inhibit cell growth. Earlier studies have been done with relatively impure preparations, however, recently, a preparation of lymphotoxin homogeneous with respect to specified criteria, and with a specific activity of the order of 10—100 million units/mg was disclosed in U.S. Appl. Serial No. 403,671 filed July 30, 1982, incorporated herein by reference, and published as European Patent Application Publication No. 0100641.

The lymphotoxin disclosed in EP 100641 is characterised by having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala.

Gamma interferon (IFN-γ) has also been shown to have anti-viral and anti-tumor activity. Preparation of IFN-γ by recombinant techniques, and its amino acid sequence were disclosed in U.S. Appl. Serial No. 312,489 filed October 19, 1981 and published as European Patent Application Publication No. 077670, April 27, 1983.

Thus, the nature and tumor inhibition activity of both of these compositions is known.

It has previously been shown that IFN-γ exhibits a synergistic effect with α- and β-interferon in assays for cell growth inhibition, as disclosed in U.S. Appl. 436,758, filed Oct. 25, 1982, corresponding to the European patent application published under No. 107,498. It has also been shown that leukocyte interferon (IFN-α) is synergistic with lymphotoxin in growth inhibition studies (see Williams, T. W. *et al., J. Immunol.*, 130:518 (1983)). On the other hand, Ware *et al, J. Immunol*, 122:1763 (1979) observed merely additive effects on cell growth from a combination of α-LT and $IF_{II}$. GB 2106117A (& DE—A—3227262) discloses target cell lysis factor (TCLF) comprising lymphotoxins. The TCLF is normally produced along with interferons, but no synergistic effect is noted, and normally the interferons are removed prior to administration. No specific formulation of a particular LT and IFN is suggested. The present invention is directed to the dramatic synergism in tumor cell inhibition which is observed when combinations of gamma interferon and a specific lymphotoxin are administered to such cells.

Summary of the invention

This invention concerns the surprising synergism of the lymphotoxin of EP 100641 and IFN-γ in cooperating to inhibit the growth of tumor cells. Accordingly in one aspect the invention is directed to a composition of matter comprising human gamma interferon (HuIFN-γ) in admixture with said human lymphotoxin (HLT) and to a process for the preparation thereof.

Brief description of the drawings

Figure 1 shows dose response curves for human lymphotoxin alone, HuIFN-γ alone, and HLT/HuIFN-γ combinations with respect to a standard assay for tumor cell growth.

Figure 2 shows the results of a comparison of treating normal cells and tumor cells with HuIFN-γ and HLT separately and in mixtures.

Detailed description

A. Definitions

Both lymphotoxin and HuIFN-γ comprise peptide sequences. Accordingly, such sequences can be found either in un-ionized form or as salts. It is recognized that when in pharmaceutical compositions or in solution, the ionization status of such molecules is entirely pH dependent. It is further recognized that such substances can be supplied in preparing compositions or solutions either as unionized materials or as their salts. Accordingly, the terms "lymphotoxin" or "HLT" and "gamma interferon" or "HuIFN-γ" refer to these organic moieties regardless of their status with respect to the ionization states of their contained amino and carboxyl groups.

"Pharmaceutically acceptable" refers to preparations which are in such form as to permit the biological activity of the active ingredients to be effective, and which contain no additional components which are toxic to the subjects to which the composition would be administered. Thus, pharmaceutically acceptable compositions or excipients refer to compositions or excipients which can reasonably be administered to a subject mammal to provide an effective dose of the active ingredient employed.

"Compositions" containing HuIFN-γ and HLT in synergistically effective amounts refers both to compositions prepared by pre-mixing these active ingredients *in vitro* and to compositions which result from tandem administration of each of these active ingredients either in solution or in admixture with excipients to a subject mammal. It is clear that the synergistic effectiveness of such compositions is independent of the manner of mixing and of the temporal mode of treatment within at least a relatively short time frame.

"Synergism" in the context of this invention is defined according to the accepted definition disclosed in Goodman, A. *et al. The Pharmacological Basis of Therapeutics*, MacMillan Publishing Co.

Inc., New York (1980). This is most easily seen in terms of the construction of an "isobologram" which plots the dosage levels required for a specific identical biological response of each of two ingredients along the X and Y axes. While simply additive effects would generate a straight line as one ingredient diminishes and the other increases, synergistic effects can be recognized by the generation of a concave curve, such that only a small increase in one component compensates for a drastic decrease in the amount of the other.

B. Demonstration of growth inhibition synergism

An isobologram demonstrating the HLT, HuIFN-γ synergism of the present invention is shown in Figure 1. Also given for comparison are results of individually administered dose levels of these components.

In the assay, human lung carcinoma A-549 cells were grown in microtiter wells at a cell density of 5,000 cells/well in a 0.2 ml volume and then exposed either to HLT or to HuIFN-γ or the combination of the two for three days. Intact cell numbers remaining were examined by staining the cells with crystal violet and assaying color intensity.

As shown in Figure 1A, 60 percent cell growth inhibition was achieved with approximately 15,000 units of human lymphotoxin. Figure 1B indicates that 60 percent inhibition would require at least 1,500 units of HuIFN-γ. However, as shown in the isobologram of Figure 1C, the combination of, for example, only 100—200 units of HuIFN-γ with 1,000 units of human lymphotoxin is sufficient to achieve 60 percent inhibition.

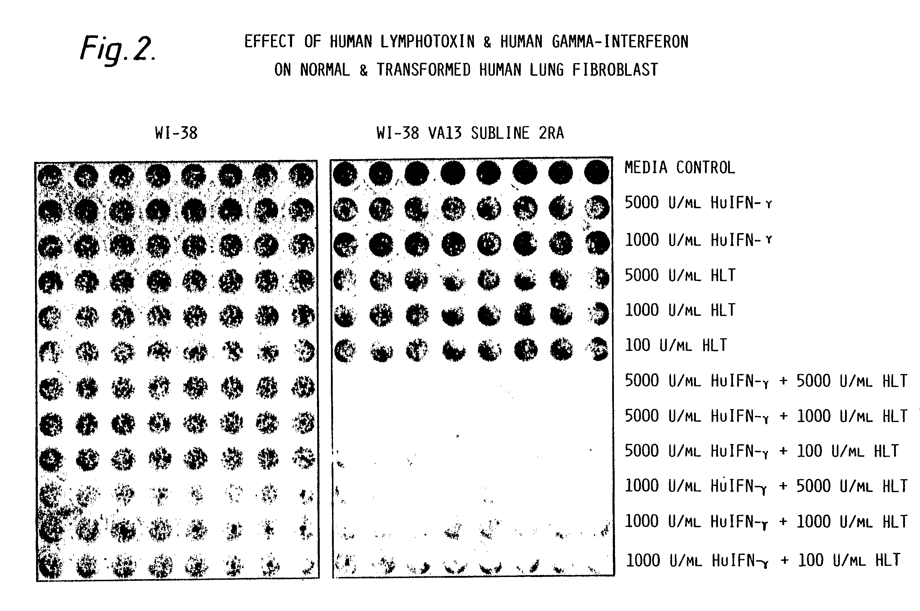

This effect is demonstrated to be unique to tumor cells in Figure 2. In that assay, normal cells were grown in the left microtiter plate and human lung fibroblast cells in the right hand microtiter plate to a level of 10,000 cells/well in 0.2 ml of medium. HuIFN-γ, HLT, or a combination of the two were added as shown and the plates incubated for 4 days. In the right hand plate, containing tumor cells, while some inhibition was achieved with HuIFN-γ or HLT alone, cell growth was completely eliminated by the combinations of the two components. For example, adding only 100 units of lymphotoxin to cells treated with 5,000 units of γ-interferon completely destroys growth, while either treatment alone permits considerable growth to take place. This effect is absent from the normal cell plate shown at the left.

Accordingly, it is clear that compositions containing both HuIFN-γ and HLT are surprisingly effective in inhibition of tumor cell growth, but relatively harmless to normal cells. Such compositions can be administered either by premixing the HuIFN-γ and HLT ingredients, or by "*in vivo*" mixing—i.e. tandem administration of each separate active ingredient.

C. Utility and administration

Administration of the compositions hereof can be via any of the accepted modes of administration for agents which exhibit such activity. These methods include oral, parenteral or topical administrations and otherwise systemic forms. Local or intravenous injection is preferred.

Depending on the intended mode of administration, the compositions used may be in the form of solid, semi-solid or liquid dosage forms, such as, for example, tablets, pills, capsules, powders, liquids, or suspensions, preferably in unit doage forms suitable for single administration of precise dosages. The compositions will include a conventional pharmaceutical carrier or excipient and, in addition, may include other medicinal agents, pharmaceuticals agents, carriers, and adjuvants. Such excipients may include other proteins, such as, for example, human serum albumin or plasma preparations.

For solid compositions, conventional nontoxic solid carriers include, for example, pharmaceutical grades or mannitol, lactose starch, or magnesium stearate. Liquid pharmaceutically administerable compositions can, for example, be prepared by dissolving, dispersing, etc. in a carrier, such as, for example, water, saline, aqueous dextrose, glycerol, and ethanol, to thereby form a solution or suspension. If desired, the pharmaceutical composition to be administered may also contain minor amounts of nontoxic auxiliary substances such as wetting or emulsifying agents, pH buffering agents and the like, for example, sodium acetate or sorbitan monolaurate. Actual methods of preparing such dosage forms are known, or will be apparent, to those skilled in this art; for example, see *Remington's Pharmaceutical Sciences*, Mack Publishing Company, Easton, Pennsylvania, 15th Edition, 1975. The composition or formulation to be administered will, in any event, contain a quantity of the active component(s) in an amount effective to achieve the desired effect in the subject being treated.

The amount of active compound administered will, of course, be dependent on the subject being treated, the severity of the affliction, the manner of administration and the judgment of the prescribing physician.

While extrapolation from *in vitro* levels to *in vivo* levels is not completely predictable, it is estimated that a suitable composition for treating a subject mammal with the composition of the invention sufficient to inhibit tumor growth would range from a combination of about 20 units of HuIFN-γ with 200 units of HLT per kg of body weight of a combination of about 100 units of HuIFN-γ with about 1,000 units of HLT per kg of body weight. However, the invention cannot be restricted to these specific ranges because of the variability in severity of affliction, and the susceptibility of the individual patient as noted above.

**Claims for the Contracting States: BE, CH, DE, FR, GB, IT, LI, LU, NL, SE**

1. A pharmaceutical preparation comprising HuIFN-γ and HLT in synergistically effective amounts and arranged for simultaneous or sequential administration, said HLT being characterised by having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala.

2. The preparation of Claim 1 wherein the active ingredients are in admixture with at least one pharmaceutically acceptable excipient.

3. The preparation of claim 1 or claim 2 wherein the HuIFNγ and HLT are admixed.

4. The preparation of claim 1 or claim 2 wherein the IFNγ and HLT are contained in separate compositions.

5. The preparation of any one of the preceding claims wherein the HuIFN-γ active ingredient is substantially free of impurities.

6. The preparation of any one of the preceding claims wherein the HLT active ingredient is substantially free of impurities.

7. The preparation of any one of the preceding claims which is free of other human lymphotoxins.

8. The preparation of claim 7 which is free of other interferons.

9. A process which comprises producing a pharmaceutical preparation of any one of the preceding claims having synergistic biological effect.

**Claims for the Contracting State: AT**

1. A process which comprises producing a pharmaceutical preparation comprising HuIFN-γ and HLT in synergistically effective amounts, and arranged for simultaneous or sequential administration said HLT being characterised by having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala.

2. The process of Claim 1 wherein the active ingredients are in admixture with at least one pharmaceutically acceptable excipient.

3. The process of claim 1 or claim 2 wherein the HuIFNγ and HLT are admixed.

4. The process of claim 1 or claim 2 wherein the IFNγ and HLT are contained in separate compositions.

5. The process of any one of the preceding claims wherein the HuIFN-γ active ingredient is substantially free of impurities.

6. The process of any one of the preceding claims wherein the HLT active ingredient is substantially free of impurities.

7. The process of any one of the preceding claims wherein the preparation is free of other human lymphotoxins.

8. The process of claim 7 wherein the preparation is free of other interferons.

**Patentansprüche für die Vertragsstaaten: BE, CH, DE, FR, GB, IT, LI, LU, NL, SE**

1. Pharmazeutische Zubereitung umfassend HuIFN-γ und HLT in synergistisch wirksamen Mengen und angeordnet für die gleichzeitige oder aufeinanderfolgende Verabreichung, wobei das genannte HLT durch die teilweise Aminosäuresequenzen His - Ser - Thr - Leu - Lys - Pro - Ala - Ala - His - Leu - Ile - Gly - Asp - Pro - Ser - Lys - Gln - Asn - und Ala - Thr - Ser - Ser - Pro - Leu - Tyr - Leu - Ala gekennzeichnet ist.

2. Zubereitung nach Anspruch 1, worin die Wirkstoffe mit wenigstens einem pharmazeutisch verträglichen Excipiens vermischt sind.

3. Zubereitung nach Anspruch 1 oder 2, worin HuIFN-γ und HLT vermischt sind.

4. Zubereitung nach Anspruch 1 oder 2, worin das IFN-γ und HLT in getrennten Zusammensetzungen enthalten sind.

5. Zubereitung nach einem der vorhergehenden Ansprüche, worin der Wirkstoff HuIFN-γ im wesentlichen frei von Verunreinigungen ist.

6. Zubereitung nach einem der vorhergehenden Ansprüche, worin der Wirkstoff HLT im wesentlichen frei von Verunreinigungen ist.

7. Zubereitung nach einem der vorhergehenden Ansprüche, die frei von anderen menschlichen Lymphotoxinen ist.

8. Zubereitung nach Anspruch 7, die frei von anderen Interferonen ist.

9. Verfahren umfassend die Herstellung einer pharmazeutischen Zubereitung nach einem der vorhergehenden Ansprüche mit synergistischer biologischer Wirkung.

**Patentansprüche für den Vertragsstaat: AT**

1. Verfahren umfassend die Herstellung einer pharmazeutischen Zubereitung, die HuIFN-γ und HLT in synergistisch wirksamen Mengen enthält und für die gleichzeitige oder aufeinanderfolgende Verabreichung angeordnet ist, wobei das genannte HLT dadurch gekennzeichnet ist, daß es die teilweisen Aminosäuresequenzen His - Ser - Thr - Leu - Lys - Pro - Ala - Ala - His - Leu - Ile - Gly - Asp - Pro - Ser - Lys - Gln - Asn - und Ala - Thr - Ser - Ser - Pro - Leu - Tyr - Leu - Ala aufweist.

2. Verfahren nach Anspruch 1, worin die Wirkstoffe mit wenigstens einem pharmazeutisch verträglichen Excipiens vermischt werden.

3. Verfahren nach Anspruch 1 oder 2, worin HuIFN-γ und HLT vermischt werden.

4. Verfahren nach Anspruch 1 oder 2, worin IFN-γ und HLT in getrennten Zusammensetzungen enthalten sind.

5. Verfahren nach einem der vorhergehenden Ansprüche, worin der Wirkstoff HuIFN-γ im wesentlichen frei von Verunreinigungen ist.

6. Verfahren nach einem der vorhergehenden Ansprüche, worin der Wirkstoff HLT im wesentlichen frei von Verunreinigungen ist.

7. Verfahren nach einem der vorhergehenden Ansprüche, worin die Zubereitung frei von anderen menschlichen Lymphotoxinen ist.

8. Verfahren nach Anspruch 7, worin die Zubereitung frei von anderen Interferonen ist.

**Revendications pour les Etats Contractants: BE, CH, DE, FR, GB, IT, LI, LU, NL, SE**

1. Préparation pharmaceutique comprenant HuIFN-γ et HLT en quantités synergiquement efficaces et agencés pour une administration simultanée ou séquentielle, HLT étant caractérisée par les séquences partielles d'acides aminés His - Ser - Thr - Leu - Lys - Pro - Ala - Ala - His - Leu - Ile - Gly - Asp - Pro - Ser - Lys - Gln - Asn - et Ala -Thr - Ser - Ser - Pro - Leu - Tyr - Leu - Ala.

2. Préparation de la revendication 1 où les ingrédients actifs sont en mélange avec au moins un excipient acceptable en pharmacie.

3. Préparation de la revendication 1 ou de la revendication 2 où HuIFN-γ et HLT sont mélangés.

4. Préparation de la revendication 1 ou de la revendication 2 où IFN-γ et HLT sont contenus dans des compositions séparées.

5. Préparation selon l'une des revendications précédentes où l'ingrédient actif HuIFN-γ est sensiblement exempt d'impuretés.

6. Préparation selon l'une des revendications précédentes où l'ingrédient actif HLT est sensiblement exempt d'impuretés.

7. Préparation selon l'une des revendications précédentes qui est exempte d'autres lymphotoxines humaines.

8. Préparation de la revendication 7 qui est exempte d'autres interférons.

9. Procédé qui comprend la production d'une préparation pharmaceutique selon l'une des revendications précédentes ayant un effet biologique synergique.

**Revendications pour l'Etat Contractant: AT**

1. Procédé qui comprend la production d'une préparation pharmaceutique comprenant HuIFN-γ et HLT en quantités synergiquement efficaces, et agencés pour une administration simultanée ou séquentielle, ladite HLT étant caractérisée par les séquences partielles d'acides aminés His - Ser - Thr - Leu - Lys - Pro - Ala - Ala - His - Leu - Ile - Gly - Asp - Pro - Ser - Lys - Gln - Asn - et Ala - Thr - Ser - Ser - Pro - Leu - Tyr - Leu - Ala.

2. Procédé de la revendication 1 où les ingrédients actifs sont en mélange avec au moins un excipient acceptable en pharmacie.

3. Procédé de la revendication 1 ou de la revendication 2 où HuIFN-γ et HLT sont mélangés.

4. Procédé selon la revendication 1 ou la revendication 2 où IFN-γ et HLT sont contenus dans des compositions séparées.

5. Procédé selon l'une des revendications précédentes où l'ingrédient actif HuIFN-γ est sensiblement exempt d'impuretés.

6. Procédé selon l'une des revendications précédentes où l'ingrédient actif HLT est sensiblement exempt d'impurtés.

7. Procédé selon l'une des revendications précédentes où la préparation est exempte d'autres lymphotoxines humaines.

8. Procédé selon la revendication 7 où la préparation est exempte d'autres interférons.

Human Lung Carcinoma, A549

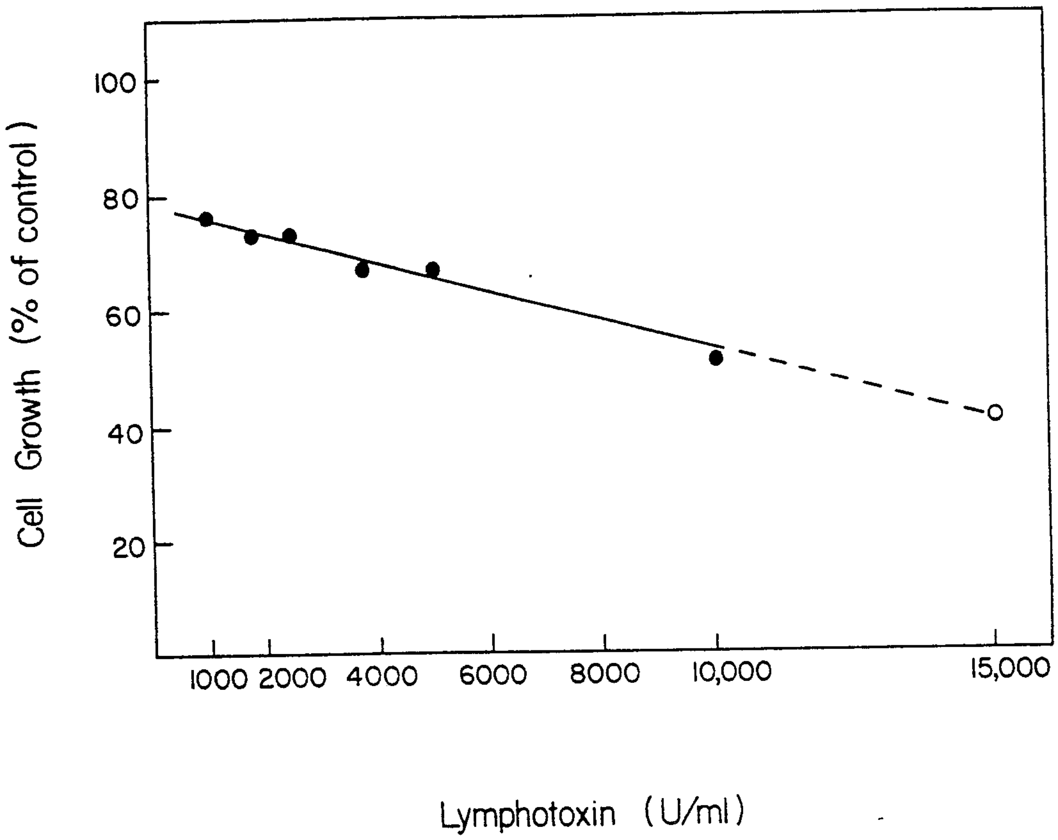


Fig.1A.

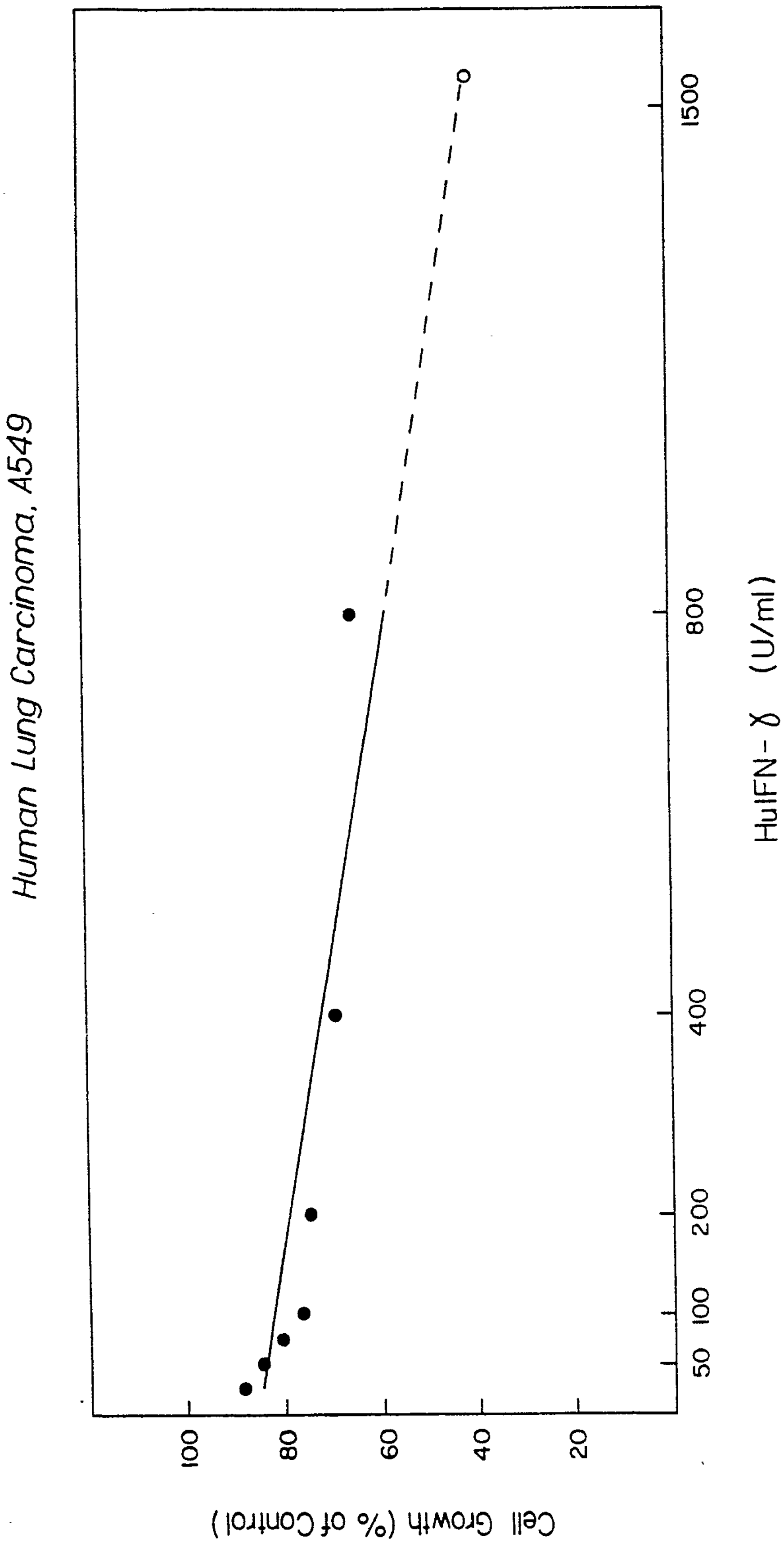
Human Lung Carcinoma, A549
Cell Growth (% of Control)
100
80
60
40
20
50
100
200
400
800
1500
HuIFN- γ (U/ml)

Fig.1B.

*Human Lung Carcinoma, A549*

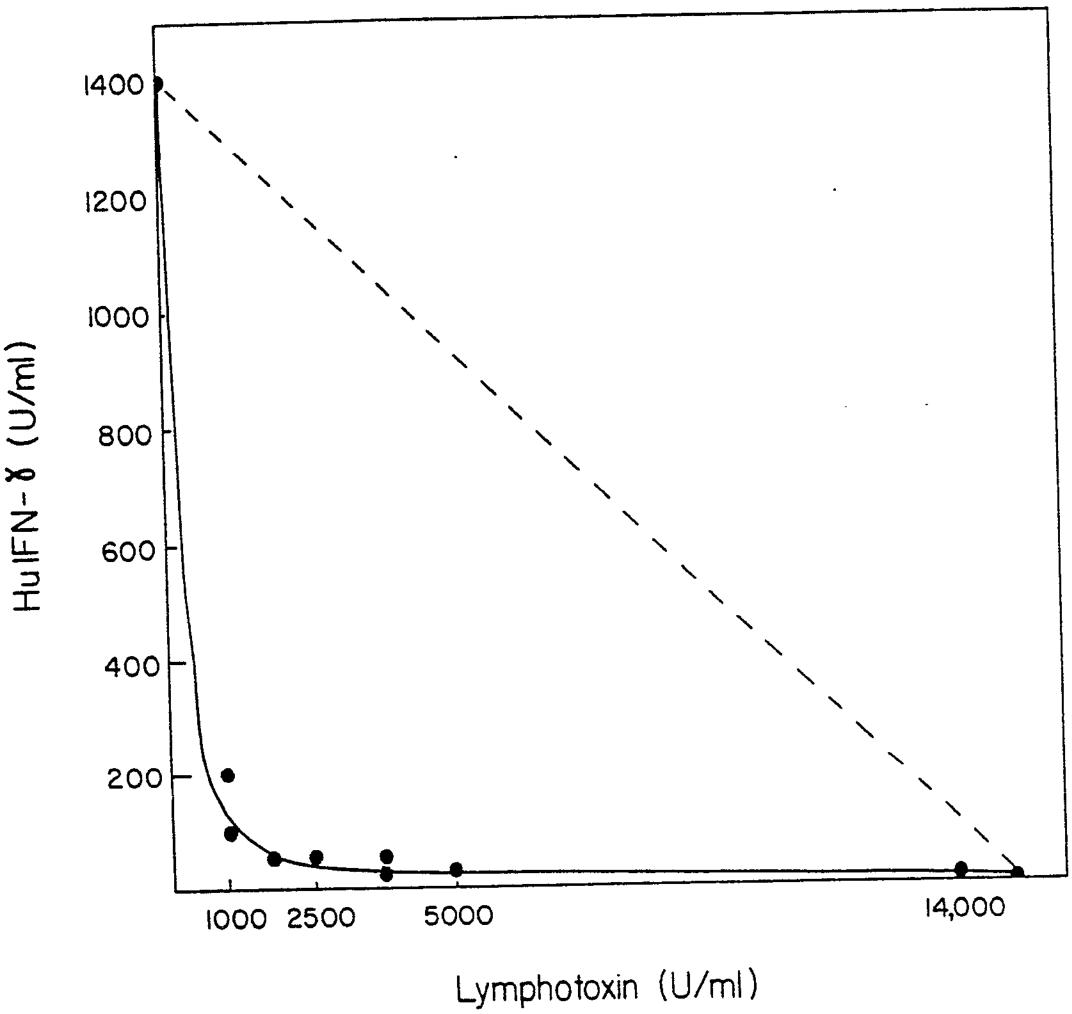


Note: Sixty Percent Inhibition Points

*Fig. 1C.*

*Fig. 2.*

EFFECT OF HUMAN LYMPHOTOXIN & HUMAN GAMMA-INTERFERON ON NORMAL & TRANSFORMED HUMAN LUNG FIBROBLAST